(19) Europäisches Patentamt
European Patent Office
Office européen des brevets

(11) EP 0 954 520 B1

(12) EUROPEAN PATENT SPECIFICATION

(45) Date of publication and mention of the grant of the patent:
**10.04.2002 Bulletin 2002/15**

(21) Application number: **98904026.6**

(22) Date of filing: **02.01.1998**

(51) Int Cl.[7]: **C07D 311/36**, A61K 31/35

(86) International application number:
**PCT/EP98/00001**

(87) International publication number:
**WO 98/29403 (09.07.1998 Gazette 1998/27)**

(54) **ISOFLAVONE DERIVATIVES, PROCESSES FOR THE PREPARATION THEREOF AND PHARMACEUTICAL COMPOSITIONS CONTAINING THEM**

ISOFLAVONDERIVATE, VERFAHREN ZU IHRER HERSTELLUNG UND DIESE ENTHALTENDE PHARMAZEUTISCHE ZUBEREITUNGEN

DERIVES DE L'ISOFLAVONE, PROCEDES POUR LES PREPARER ET COMPOSITIONS PHARMACEUTIQUES LES CONTENANT

(84) Designated Contracting States:
**AT BE CH DE DK ES FI FR GB GR IE IT LI LU MC NL PT SE**
Designated Extension States:
**AL LT LV MK RO SI**

(30) Priority: **03.01.1997 IT MI970003**

(43) Date of publication of application:
**10.11.1999 Bulletin 1999/45**

(73) Proprietor: **CHIESI FARMACEUTICI S.p.A.**
**I-43100 Parma (IT)**

(72) Inventors:
- **CHIESI, Paolo**
  **I-43100 Parma (IT)**
- **VENTURA, Paolo**
  **I-43100 Parma (IT)**
- **SERVADIO, Vittorino**
  **I-43100 Parma (IT)**
- **DELCANALE, Maurizio**
  **I-43100 Parma (IT)**
- **AMARI, Gabriele**
  **I-43100 Parma (IT)**
- **ARMANI, Elisabetta**
  **I-43100 Parma (IT)**
- **CIVELLI, Maurizio**
  **I-43100 Parma (IT)**
- **GIOSSI, Massimo**
  **I-43100 Parma (IT)**
- **GALBIATTI, Elisabetta**
  **I-43100 Parma (IT)**

(74) Representative: **Minoja, Fabrizio, Dr. et al**
**Bianchetti Bracco Minoja S.r.l.**
**Via Rossini, 8**
**20122 Milano (IT)**

(56) References cited:
**WO-A-95/03040**
**WO-A-95/03293**
**FR-A- 1 320 300**
**GB-A- 1 360 461**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 954 520 B1

## Description

The present invention relates to isoflavone derivatives, processes for the preparation thereof and pharmaceutical compositions containing them.

More precisely, the invention relates to isoflavone derivatives of general formula:

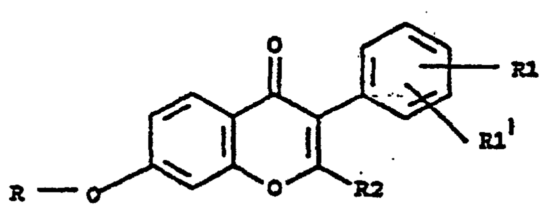

wherein R is hydrogen or optionally substituted straight or branched $C_1$-$C_6$ alkyl;
$R_1$ is hydrogen, hydroxyl, halogen, straight or branched $C_1$-$C_6$ alkyl, $C_3$-$C_6$ cycloalkyl, cycloalkylalkyl, $C_1$-$C_6$ alkoxy, a trifluoromethyl or trifluoromethoxyl group;
$R_1$' is hydrogen, hydroxyl, halogen, $C_1$-$C_4$ alkyl or $C_1$-$C_4$ alkoxy;
$R_2$ is a p-hydroxybenzoyl group wherein the hydroxyl can be substituted with alkyl, alkanoyl, N,N-dialkylaminoalkyl or with a piperidinoalkyl, pyrrolidinoalkyl, morpholinoalkyl, piperazinoalkyl residue, optionally substituted in its turn at the 4- position, when possible.

Preferably, $R_1$' is hydrogen and at least one of the 'groups $R_1$ or -OR is hydroxy.

When not otherwise stated, "alkyl, alkoxy, alkanoyl or aminoalkyl" mean an alkyl residue containing 1 to 4 carbon atoms.

The compounds of the invention can exist in the form of salts.

The compounds containing basic groups can exist in the form of organic or inorganic acid addition salts.

The compounds containing acid groups can exist in the form of addition salts with alkali or alkaline-earth metal or organic amines.

When R is hydrogen and/or $R_1$, $R'_1$ is hydroxyl, the compounds of the invention can exist in the form of esters.

The compounds of the invention are provided with a specific pharmacological activity for the bone tissue which performs both through an inhibition of bone resorption and, in part, through a stimulation of the processes of bone formation and therefore can be used for the prophylaxis and treatment of osteoporosis.

STATE OF THE ART

Osteoporosis is a disease condition characterized by a reduction in bone mass leading to increased skeletal fragility and fractures

Natural and synthetic compounds having a isoflavone structure are endowed with various pharmacological activities: some of them have estrogenic activity, others anti-estrogenic activity; some were described as antihypertensives, coronary-dilators or antihyperlipemics; others as potential bronchodilators or antiinflammatories or antispasmodics; others still as anticonvulsants.

See for example J Med Chem 9, 979, 1966; J Med Chem 10, 154, 1967; Acta Pharm Hung 38, 252, 1968; Acta Pharm Hung 44, 1, 1976; Japanese Patent applications JP 7232074 (C.A. 78:4125); JP 7248387 (C.A. 78:97483); JP 7310076 (C.A. 78:124448) (Sakai); French Patent n° FR 1320300 (Cassella); Belgian Patent n° BE 666541 (Siphar).

Some isoflavone compounds have antiosteoporotic action.

One of them, 7-isopropoxy-isoflavone or ipriflavone, has been used for a long time in the prevention and treatment of post-menopausal and senile osteoporosis.

Ipriflavone and structural analogues have been described in GB patents n° 1360461, n° 1360462, n° 1434451 and n° 1482238 (Chinoin) as well as in EP-A2-0135172, EP-A2-136569, EP 146921 and EP 146922 (Takeda).

Other isoflavones have subsequently been disclosed in PCT n° WO 95/03293 and WO 95/03040 (Chinoin).

According to the present invention new derivatives of isoflavone, containing a 4-hydroxybenzoyl residue in position 2, have now been identified.

These new compounds have a good activity in inhibiting bone resorption and have the advantage over previously described ipriflavone and analogue compounds that are also provided with a tissue-specific affinity for the estrogen receptors.

By this property they have the beneficial effects of estrogen on bone but don't have uterotrophic effects.

EXAMPLE 1. Preparation of 7-hydroxy-4'-methoxy-2-(4-(2-(N-piperidinyl)ethyloxy)benzoyl)isoflavone hydrochloride (CHF 3290.01)

a) Preparation of 4-methoxyphenylacetic acid chloride.

A mixture of 20 g of 4-methoxyphenylacetic acid and 20 g of thionyl chloride is refluxed for 30 min. then evaporated at 80°C. 21.5 g of an orange fluid oil are obtained.

b) Preparation of 2.4-dihydroxyphenyl-4'-methoxyben zylketone

A mixture of 22 g of aluminium trichloride in 250 ml of 1,2-dichloroethane is added with 22 g of resorcin, then cooled to 10°C and added with a solution of 22 g of 4-methoxyphenylacetic acid chloride in 50 ml of 1,2-dichloroethane, in 30 min, under mechanical stirring.

Stirring is continued at r.t. for 1 hour, then the mixture is poured into 300 g of ice, added with 200 ml of 1,2-dichloroethane and 700 ml of chloroform, then with 500 ml of 37% HCl and heated until complete dissolution of the suspended solid. The organic phase is separated, dried over sodium sulfate, evaporated to dryness and the residue is treated with 50 ml of cold toluene, filtered, washed with hexane and dried under vacuum at 40°C. 21.1 g of a white solid are obtained.

c) Preparation of 4-(2-bromoethyloxy)-benzoylformic acid

A suspension of 40 g of aluminium trichloride in 700 ml of chloroform is added with 17 ml of ethyloxalyl chloride and 30 g of 2-bromophenetole dissolved in 200 ml of chloroform, under stirring.

The mixture is stirred for 3 h at r.t., then poured into 700 g of ice and added with 400 ml of 37% HCl.

The organic phase is separated, dried over sodium sulfate and evaporated under vacuum.

The oily residue (ethyl 4-(2-bromoethoxy)-benzoylformate) is dissolved in 300 ml of ethanol and treated with 75 ml of 2N NaOH for 30 min. The mixture is evaporated under vacuum, taken up with 600 ml of water and 300 ml of ethyl acetate.

The aqueous phase is separated, acidified with 10% HCl and extracted with 2x300 ml of ethyl acetate.

The organic phases are dried over sodium sulfate and evaporated under vacuum to give an oil which solidifies upon stirring in 200 ml of petroleum ether. The slightly pink solid is filtered and dried under vacuum at 25°C. 25 g of the product are obtained.

d) Preparation of 2-(4-(2-bromoethyloxy)benzoyl)-7-hydroxy-4'-methoxy isoflavone

15.8 g of the acid from step c) are added with 5.2 ml of dichloromethyl-methyl ether. The mixture, protected with a calcium chloride valve, is stirred at r.t. for 20 min, then at 60°C for 60 min and then evaporated under vacuum at r.t. for 15 min.

The resulting 4-(2-bromoethyloxy)-benzoylformyl chloride is dropped immediately in less than 10 min, into a mixture of 7 g of the product from step b) in 35 ml of pyridine dry, stirred magnetically and cooled at 0°C.

After 48 hours at r.t., the dark red solution is poured into 500 ml of water, acidified with 10% HCl and extracted with 2x400 ml of ethyl acetate. The organic phases are washed with 300 ml of 10% HCl, dried over sodium sulfate and evaporated under vacuum to give 15 g of dark red residue. The residue is taken up in 45 ml of glacial acetic acid, added with 9 ml of 37% HCl and the mixture is heated under stirring at 100°C for 1 hour and 30 min., then poured into 300 ml of water and extracted with 2x300 ml of ethyl acetate.

The organic phases are washed with 2x300 ml of a bicarbonate saturated solution and with water to neutrality. The organic phase is dried over sodium sulfate and evaporated under vacuum to give an orange residue which solidifies when treated grinding in 300 ml of ethyl ether. The yellow ochre solid is filtered and dried under vacuum at 40°C. 1 g of product is obtained.

e) Preparation of 7-hydroxy-4'-methoxy-2-(4-(2-(N-piperidinyl) ethyloxy)benzoyl) isoflavone hydrochloide (CHF 3290.01)

400 mg of 2-(4-(2-bromoethyloxy)benzoyl-7-hydroxy-4'-methoxy isoflavone and 400 mg of piperidine are heated to 80°C for 45 min. in a closed vessel.

The mixture is taken up into 100 ml of methanol, and the solution is evaporated under vacuum. The residue is dissolved in ethyl acetate ÷ $CH_2Cl_2$ (300 ml÷30 ml) and washed with 200 ml of a bicarbonate solution and with water. The organic phase is dried over sodium sulfate and evaporated under vacuum and the residue is purified by flash chromatography ($CH_2Cl_2$:MeOH = 94:6). The suitable fractions are evaporated under vacuum, the residue is taken up with methanol, acidified with methanolic HCl and evaporated under vacuum. 125 mg of the hydrochloride product as a pale yellow solid are obtained.

EXAMPLE 2. Preparation of 4',7-dihydroxy-2-(4-(2-(N-piperidinyl) ethyloxy) benzoyl)isoflavone hydrochloride (CHF 3316.01)

A solution of 400 mg of the compound obtained according to example 1, used in the form of free base, in 60 ml of methylene chloride, is added with 4.5 ml of 1M $BBr_3$ in methylene chloride at r.t. under stirring. After 3 hours the solid and the tarry residues are separated and dissolved in the minimum amount of methanol.

The solution is added with 200 ml of ethyl acetate and 200 ml of a bicarbonate solution; the mixture is stirred for 2 hours, then the phases are separated, extracting the aqueous phase with a further 200 ml of ethyl acetate.

The organic phases are dried over sodium sulfate and evaporated under vacuum and the residue is subjected to flash chromatography ($CH_2Cl_2$:MeOH = 95:5). The suitable fractions are combined and evaporated under vacuum to give a tarry solid which is dissolved in 50 ml of acetone and acidified with 37% HCl. Evaporation-under vacuum gives 100 mg of the hydrochloride product as a pale yellow solid.

EXAMPLE 3. Preparation of 7-hydroxy-4'-methoxy-2-(4-(2-(1-piperazinyl)ethyloxy)benzoyl)isoflavone dihydrochloride (CHF 3340.01)

300 mg of 2-(4-(2-bromoethyloxy)benzoyl)-7-hydroxy-4'-methoxy isoflavone, prepared as described in example 1.d), and 350 mg of 1-acetylpiperazine are heated at 80°C for 45 min.

1.5 ml of 37% HCl are added and the mixture is refluxed for 20 min.

After evaporation under vacuum, the crude material is submitted to flash-chromathography ($CH_2Cl_2$:MeOH=80:20) giving 150 mg of the title compound.

EXAMPLE 4. Preparation of 4',7-dihydroxo-2-(4-(2-(1-piperazinyl)ethyloxy)benzoyl)isoflavone dihydrochloride (CHF 3356.01)

The product is obtained using CHF 3340.01 as reagent and operating as described in example 2.

The compounds prepared are listed in Table 1.

Table 1

| CHF | Structure | Molecular Formula | M. W. | Melting Point |
|---|---|---|---|---|
| 3290,01 | 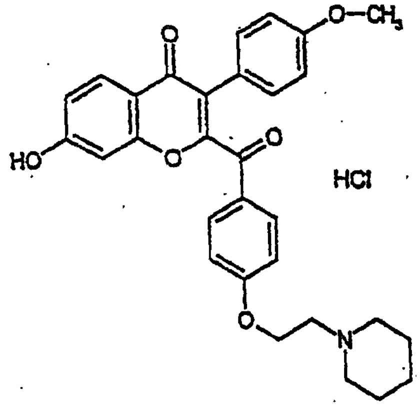 HCl | C30H30ClNO6 | 536,03 | |
| 3316,01 | 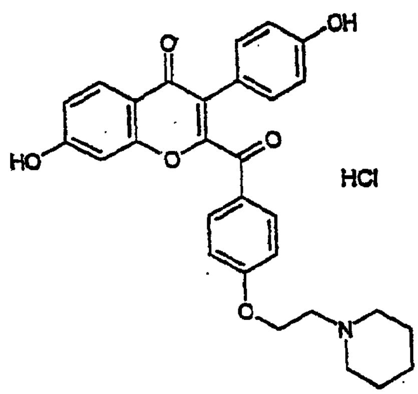 HCl | C29H28ClNO6 | 522,00 | 218-222 |
| 3340,01 | 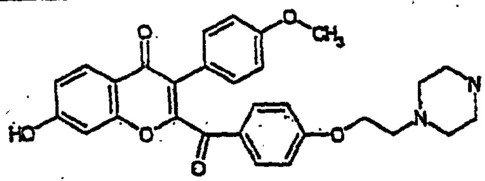 2 HCl | C29H30Cl2N2O6 | 573,48 | |
| 3356,01 | 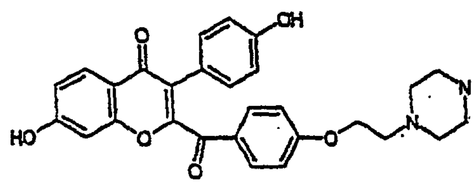 2 HCl | C28H28Cl2N2O6 | 559,45 | |

Bone resorption in vitro

Effect of CHF 3316.01 on bone resorption in rat long bone culture

To assess the in vitro activity of the compounds of the invention on bone resorption as a marker of a potential antiosteoporotic effect osteoclastic bone resorption was assayed using fetal rat long bones (radius and ulna) in stationary cultures, where resorption induced by bovine parathyroid hormone fragment 1-34 (bPTH 1-34) was measured by assaying the release from bones of previously incorporated 45Ca (DeLean A et al Am. J. Physiol., (1978), 225, E97-E102).

To validate the experimental conditions adopted, the activity of the well-known resorption inhibitor dichloromethylene bisphosphonate (disodium clodronate) was determined.

Pregnant Sprague-Dawley rats were injected subcutaneously with 0.2 mCi 45Ca ($CaCl_2$, >10 Ci/g) on the 18th day of gestation (day of vaginal plug discovery equals day 0 of pregnancy).

On the following day the animals were sacrificed, the fetuses removed and the radius and ulna aseptically excised and dissected free of surrounding soft tissues.

To remove freely exchangeable 45Ca, bones were pre-incubated for 24 hours in 48-well polystyrene culture dishes (Costar, Cambridge, MA) containing 500 µl of a culture medium (BGJ-B medium) in an air humidified incubator at 37°C in 5% C02.

Bones were then cultured for 5 days in the culture medium containing 1 mg/ml bovine serum albumine in the absence or in the continuous presence of bPTH 1-34 5 nM, CHF 3316.01 at 3, 10 and 30 µM and disodium clodronate 5 µM. The medium was changed after 2 days.

The experiment was terminated by washing the bones in 500 µl distilled water and extracting the residual radioactivity with 500 µl of 10% trichloroacetic acid (TCA).

bPTH 1-34 and disodium clodronate were dissolved in the culture medium.

CHF 3316.01 was dissolved in dimethylsulfoxide (DMSO) and added to a DMSO concentration of 0.1%. This amount of DMSO was added to control and to stimulated cultures and did not affect resorption.

In our experimental conditions the unstimulated control cultures released 6.06±1.13% (n= 7) of the total bone 45Ca by day 5.

Exposure of fetal bones to 5 nM bPTH 1-34 produced a significant rise in 45Ca release on day 5, resulting in treated over control (T/C) ratio of 4.27±0.33 (n=7). This concentration of bPTH 1-34 was the lowest producing maximal effect and was chosen on the basis of preliminary dose-response experiments.

Disodium clodronate 5 µM gave 90±5% inhibition of resorption (n = 3 bones).

CHF 3316.01 significantly reduced 45Ca release induced by bPTH producing 24.81±2.14%, 37.86±3.73% and full inhibition at 3, 10 and 30 mM respectively. The apparent IC50 value was 10 mM. The test compound appeared about 5 fold more potent than the metabolite 2 of ipriflavone M2 (4',7-dihydroxyisoflavone) and 2 fold than the metaboite 3 M3 (4'-hydroxy-7-[(1-methyl) ethoxy]isoflavone), the most active metabolite of ipriflavone, so eliciting an evident antiresorptive in vitro activity.

**Estrogen receptor binding affinity**

To determine the estrogen receptor binding affinity of CHF 3316.01 calf uterus, prepared as previously described in McGuire W. L. et al. Eds. Estrogen receptors in human breast cancer, Raven Press N. Y., (1975), was used as source of estrogen receptors.

Displacement curves were generated using final concentrations of 1.5 nM 17b-[3H]estradiol as radioligand and $\frac{1}{2}$-log unit increments of CHF 3316.01, ranging from 0.1 to 10,000 nM.

Incubation time was 24 hr at 4°C, after which dextran-treated charcoal was added. The suspension was then centrifuged, and radioactivity in the supernatant fluid was determined by scintillation counting. Percent binding was determined in duplicate at each concentration of displacing ligand.

Non-specific binding was defined as that which occurred in the presence of 5.8 mM diethylstilbestrol and represented ~ 20% of the total binding.

$IC_{50}$ value for 50 % inhibition of 17b-[3H]estradiol binding and Ki value were determined by non linear regression analysis of log concentration-displacement curves.

CHF 3316.01 inhibited the specific binding of 17b-[3H]estradiol to a single population of binding sites in calf uterus with a Ki value of 3.9±0.3 nM. The test compound is a higher affinity estrogen receptor ligand than the structurally related compound M2 (metabolite 2 of ipriflavone) (Ki = 127 nM) and the well-known selective estrogen receptor modulator tamoxifene (Ki = 6.8 nM).

Uterotrophic and antiuterotrophic actions of CHF 3316.01

The uterotrophic and antiuterotrophic actions of CHF 3316.01 were studied to determine the estrogenic and antiestrogenic activity of CHF 3316.01 in immature rat assay.

All experiments were conducted using subcutaneous injection of the test compound as a solution in 20% Solutol HS 15. Each experiment was accompanied by a vehicle control group and a positive control group (17b-estradiol 2 mg/kg by subcutaneous injection).

The test protocol was based on the immature rat assay protocol described by Wakeling A.E. and Valcaccia B. J Endocr (1978), 99, 445-464.

Animals were dosed on 3 successive days with the appropriate material. Animals were killed by an overdose of $CO_2$ 24 hr after the final dose. The body of the uterus was cut just above its junction with the cervix and at the junction of the uterine horns with the ovaries. The uterus was then weighed. Generally, 6 animals per exposure group were employed.

The daily dose levels used for CHF 3316.01 were 10 and 20 mg/kg.

Three successive daily injections of 17b-estradiol (2 mg/kg) significantly increased uterine weight in immature rats (93% increase above vehicle control group). The isoflavone derivative of the invention, CHF 3316.01, inhibited the uterotrophic action of estradiol in a dose response fashion. In fact, at 10 and 20 mg/Kg per day, the compound suppressed 63 and 78% of estradiol's activity, respectively.

At the same dose levels, CHF 3316.01 was found to be devoid of uterotrophic activity.

The compounds of formula (I) can be used for the preparation of solid or liquid pharmaceutical compositions, in admixture with pharmaceutically acceptable excipients, according to the conventional techniques.

Preferred pharmaceutical compositions are those which can be administered orally, particularly those in the form of tablets or capsules, optionally sustained-release forms.

The daily dosage for an adult will range from 10 to 500 mg.

## Claims

1. Isoflavone derivatives of general formula:

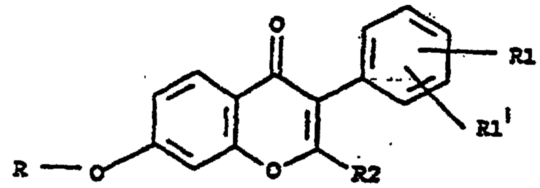

wherein R is hydrogen or optionally substituted straight or branched $C_1$-$C_6$ alkyl;
$R_1$ is hydrogen, hydroxyl, halogen, straight or branched $C_1$-$C_6$ alkyl, $C_3$-$C_6$ cycloalkyl, cycloalkylalkyl, $C_1$-$C_6$ alkoxy, a trifluoromethyl or trifluoromethoxyl group;
$R_1$' is hydrogen, hydroxyl, halogen, $C_1$-$C_4$ alkyl or $C_1$-$C_4$ alkoxy;
$R_2$ is a p-hydroxybenzoyl group wherein the hydroxyl can be substituted with alkyl, alkanoyl, N,N-dialkylaminoalkyl or with a piperidinoalkyl, pyrrolidinoalkyl, morpholinoalkyl, piperazinoalkyl residue, optionally substituted in its turn at the 4- position, when possible;

optionally in form of pharmaceutically acceptable salts or esters.

2. A compound of formula 1 wherein R and $R_1$' are hydrogen, $R_1$ is hydroxyl and $R_2$ is 4-(2-(N-piperidinyl)ethyloxy) benzoyl.

3. Pharmaceutical compositions containing as the active ingredient at least one compound as claimed in claims 1 and 2.

4. The use of the compounds according to claims 1-2 for the preparation of a medicament useful in the prevention and treatment of osteoporosis.

## Patentansprüche

1. Isoflavon-Derivate der allgemeinen Formel:

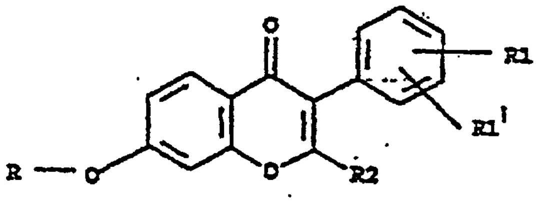

wobei R Wasserstoff oder gegebenenfalls substituiertes geradkettiges oder verzweigtes $C_1$-$C_6$-Alkyl ist;
$R_1$ Wasserstoff, Hydroxyl, Halogen, geradkettiges oder verzweigtes $C_1$-$C_6$-Alkyl, $C_3$-$C_6$-Cycloalkyl, Cycloalkylalkyl, $C_1$-$C_6$-Alkoxy, eine Trifluoromethyloder Trifluoromethoxygruppe ist;
$R_1$' Wasserstoff, Hydroxyl, Halogen, $C_1$-$C_4$-Alkyl oder $C_1$-$C_4$-Alkoxy ist;
$R_2$ eine p-Hydroxybenzoylgruppe ist, wobei das Hydroxyl mit Alkyl, Alkanoyl, N,N-Dialkylaminoalkyl oder mit einem Piperidinoalkyl-, Pyrrolidinoalkyl-, Morpholinoalkyl-, Piperazinoalkylrest substituiert sein kann, der gegebenenfalls seinerseits an der 4-Position substituiert ist, falls möglich;

gegebenenfalls in Form von pharmazeutisch verträglichen Salzen oder Estern.

2. Verbindung der Formel 1, wobei R und $R_1$' Wasserstoff sind, $R_1$ Hydroxyl ist und $R_2$ 4-(2-(N-Piperidinyl)-ethyloxy) benzoyl ist.

3. Pharmazeutische Zusammensetzungen, enthaltend als aktiven Bestandteil mindestens eine Verbindung wie in den Ansprüchen 1 und 2 beansprucht.

4. Verwendung der Verbindungen nach den Ansprüchen 1 bis 2 zur Herstellung eines Medikaments, das bei der Vorbeugung und Behandlung von Osteoporose einsetzbar ist.

## Revendications

1. Dérivés d'isoflavone de formule générale :

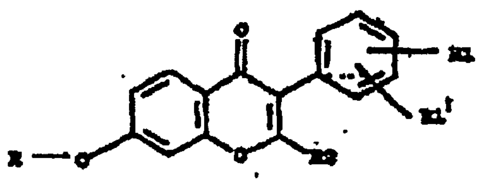

dans laquelle R est un hydrogène ou un groupe alkyle en $C_1$ à $C_6$ linéaire ou ramifié éventuellement substitué ;
$R_1$ est un hydrogène, un groupe hydroxyle, un halogène, un groupe alkyle en $C_1$ à $C_6$ linéaire ou ramifié, cycloalkyle en $C_3$ à $C_6$, cycloalkylalkyle, alcoxy en $C_1$ à $C_6$, trifluorométhyle ou trifluorométhoxyle ;
$R_1$' est un hydrogène, un groupe hydroxyle, un halogène, un groupe alkyle en $C_1$ à $C_4$ ou alcoxy en $C_1$ à $C_4$;
$R_2$ est un groupe p-hydroxybenzoyle dans lequel l'hydroxyle peut être substitué par un groupe alkyle, alcanoyle, N,N-dialkylaminoalkyle ou avec un résidu pipéridinoalkyle, pyrrolidinoalkyle, morpholinoalkyle, pipérazinoalkyle, éventuellement substitué à son tour en position 4, lorsque cela est possible ;

éventuellement sous la forme de sels ou esters pharmaceutiquement acceptables.

2. Composé de formule 1, dans lequel R et $R_1$' sont de l'hydrogène, $R_1$ est un groupe hydroxyle et $R_2$ est un groupe 4-(2-(N-pipéridinyl)éthoxy)benzoyle.

**3.** Compositions pharmaceutiques contenant en tant qu'ingrédient actif au moins un composé tel que revendiqué dans les revendications 1 et 2.

**4.** Utilisation des composés selon les revendications 1 à 2 pour la préparation d'un médicament utile dans la prévention et le traitement de l'ostéoporose.